# EUROPEAN PATENT APPLICATION

(11) **EP 3 766 542 A1**
(43) Date of publication of application: **20.01.2021**
(21) Application number: 19717959.1
(22) Date of filing: 26.02.2019
(51) Int. Cl.: A61P 15/08, A61K 9/00, A61K 41/00, C12N 5/075, C12N 15/873, G01N 33/68, A61K 47/69

(54) **MAGNETIC NANOPARTICLES FOR USE IN ASSISTED REPRODUCTION**

(30) Priority: 11.03.2018 ES 201830239
(71) Applicant: Universidad de Murcia, 30100 Murcia (ES)
(72) Inventor: GARCIA VAZQUEZ, Francisco Alberto, 30100 Murcia (ES); JIMENEZ MOVILLA, Maria, 30100 Murcia (ES); CABALLERO SASTRE, Maria, 30100 Murcia (ES)
(74) Representative: Herrero & Asociados, S.L.
(86) International application number: PCT/ES2019/070108
(87) International publication number: WO 2019/175449

(57) **Abstract**

The present invention relates to a method of preparing magnetic oocytes and/or embryos by means of nanoparticles for assisted reproduction techniques and to the use in assisted reproduction techniques of non-human oocytes and/or embryos prepared with said method. Said method comprises: a step in which magnetic nanoparticles are conjugated with oviduct recombinant protein, OVGP1r; a step in which it is verified that the nanoparticle-OVGP1r conjugation attaches to the ZP of oocytes/embryos after being incubated together; and a step in which the number of oocytes/embryos having nanoparticles attached to them distributed around the ZP without being endocytosed is verified; and it is assessed if the amount of magnetic nanoparticles attached to the ZP of oocytes/embryos is enough to be attracted by a magnetic field.

## Description

### Object of the Invention

As expressed in the title of the present specification, the invention relates to a method of preparing magnetic oocytes and/or embryos by means of nanoparticles for assisted reproduction techniques and to the use in assisted reproduction techniques of non-human oocytes and/or embryos prepared with said method, which is a significant novelty in the current state of the art in its field of application.

More particularly, the object of the invention relates, on one hand, to a method for the magnetization of oocytes and/or embryos based on the use of magnetic nanoparticles and a protein attachment technology for attaching the nanoparticles and the outer part of the oocytes/embryos, referred to as zona pellucida (ZP), since said magnetization of oocytes/embryos may become a very useful property in the use of such oocytes/embryos in different assisted reproduction techniques in different animal species.

Essentially, the process comprises a first step in which the magnetic nanoparticles are conjugated with the recombinant oviductal fluid protein, oviduct (OVGP1r); a second step in which said nanoparticle-OVGPlr conjugation, in turn, attaches to the ZP of oocytes/embryos by means of being incubated together or co-incubation; and a third step in which, it is assessed, by means of a magnetic field, that the amount of magnetic nanoparticles attached to the ZP of oocytes/embryos is enough for them to be attracted by a magnetic field.

Likewise, a second aspect of the invention relates to the specific use in assisted reproduction techniques of non-human oocytes and/or embryos prepared with said method.

### Field of Application of the Invention

The field of application of the present invention is comprised in the biochemistry, nanobiotechnology, or nanomedicine sectors, specifically in the field of assisted reproduction techniques.

### Background of the Invention

The use of magnetic nanoparticles is very widespread today in the field of biotechnology and biomedicine since it is a non-invasive procedure for diagnosis and therapy of some diseases, such as cancer, Alzheimer's disease, or in stem cell transplant in diseases such as myocardial infarction. In the branch of assisted reproduction, these magnetic nanoparticles are currently used in sperm cells, on one hand, for the detection of spermatozoa damaged when said nanoparticles are conjugated with different antibodies and lectins which attach to said gametes, and on the other hand, for use thereof in sperm-mediated gene transfer.

However, the present invention relates to the use of magnetic nanoparticles in oocytes and/or embryos through a novel protein attachment technology for attaching the nanoparticles conjugated with recombinant protein OVGP1r and the outer part of the oocytes/embryos, referred to as zona pellucida (ZP), making said attachment very specific. Logically, the OVGP1 protein present in oviductal fluid attaches to the ZP of oocytes and embryos.

As a reference to the current state of the art, it should be noted that patents and documents relating to the subject matter of the present invention are known, and the most relevant ones are:
Patent document EP2237039A1, which relates to an encoded microparticle of a biocompatible material for tagging and/or tracking an isolated oocyte or an isolated embryo. Said microparticle is fixed to the ZP of the cell and can be tracked, by means of optical microscopy, due to its outer shape, which constitutes a particular identification code.

Patent document US2016091410A1 discloses a method for processing animal sperm to improve their motility, viability, and fertility, comprising compositions with magnetic particles.

The document entitled *"*Barcode tagging of human oocytes and embryos to prevent mix-ups in assisted reproduction technologies" published in 2014, by Novo Sergi; Nogues Carme; Penon Oriol; Barrios Leonardo; Santalo Josep; Gomez-Martinez Rodrigo; Esteve Jaume; Errachid Abdelhamid; Antonio Plaza Jose; Perez-Garcia Lluisa; Ibanez Elena, describes the application in assisted reproduction technologies of tagged human oocytes and embryos in the zona pellucida with biofunctionalized polysilicon barcodes.

The document entitled *"*Direct embryo tagging and identification system by attachment of biofunctionalized polysilicon barcodes to the zona pellucida of mouse embryos", published in 2013, by Novo Sergi; Penon Oriol; Barrios Leonardo; Nogues Carme; Santalo Josep; Duran Sara; Gomez-Matinez Rodrigo; Samitier Josep; Antonio Plaza Jose; Perez-Garcia Luisa; Ibanez Elena, describes a direct embryo tagging and identification system by attachment of biofunctionalized polysilicon barcodes to the zona pellucida of mouse embryos.

The document entitled *"*Biomolecule screening for efficient attachment of biofunctionalized microparticles to the zona pellucida of mammalian oocytes and embryos", published in 2013, by Novo S; Ibañez E; Barrios L; Castell O; Nogues C, describes the biomolecule screening for efficient attachment of biofunctionalized microparticles to the ZP of mammalian oocytes and embryos. The individual tagging of oocytes and embryos by attaching polysilicon barcodes to the ZP is a technique which is applied in human assisted reproduction and in animal production programs. To provide barcodes capable of attachment to the ZP, they must first be subjected to a biofunctionalization process by means of the conjugation thereof to a biomolecule capable of attachment to the ZP of oocytes and embryos. The analyzed biomolecules are anti-ZP2 antibody, two lectins, wheat germ agglutinin (WGA) and phytohemagglutinin-1.

The document entitled *"*Bioluminescent magnetic nanoparticles as potential imaging agents for mammalian spermatozoa", published in 2016, by Vasquez E S; Feugang J M; Willard S T; Ryan P L; Walters K B, describes bioluminescent magnetic nanoparticles as potential imaging agents for mammalian spermatozoa. Bioluminescent compounds comprising magnetic nanoparticles and firefly (*Photinus pyralis*) luciferase are analyzed as potential light-emitting agents for image projection, and mammalian sperm detection and monitoring.

The document entitled *"*Lectin-Functionalized Magnetic Iron Oxide Nanoparticles for Reproductive Improvement", published in 2015, by Feugang J M; Shengfa F L; Crenshaw M A; Clemente H; Willard S T; Ryan P L, describes the use of lectin-biofunctionalized magnetic iron oxide nanoparticles as a new sperm cell purification and screening strategy to increase the fertility of semen in assisted reproduction methods.

The document entitled "The C-terminal region of OVGP1 remodels the zona pellucida and modifies fertility parameters", published in 2016, by Algarra B; Han L; Soriano-Ubeda C; Aviles M; Coy P; Jovine L; Jimenez-Movilla M., analyzes the function of the C-terminal region of OVGP1 ('Oviduct-specific glycoprotein 1') protein in the process of OVGP1 attachment to the extracellular zona pellucida of the oocyte and in protein activity during fertilization.

Nevertheless, it can be seen that none of the aforementioned patents or documents describes, when considered separately or combined with one another, the present invention, as it is claimed.

### Description of the Invention

The method of preparing magnetic oocytes and/or embryos by means of nanoparticles for assisted reproduction techniques and use in assisted reproduction techniques of non-human oocytes and/or embryos prepared with said method proposed by the invention is configured as a significant novelty within its field of application, and the characterizing details which distinguish them are suitably described in the final claims enclosed with the present description.

As previously indicated, the invention proposes a method for the magnetization of oocytes and/or embryos of different animal species based on the use of magnetic nanoparticles conjugated with OVGP1 protein present in oviductal fluid and in this case expressed in a recombinant manner (OVGP1r) which exhibits affinity for the outer part of the oocytes/embryos, referred to as zona pellucida (ZP), which provides a very useful property in handling such oocytes/embryos in different assisted reproduction techniques.

Specifically, the method proposed by the invention comprises at least the following steps:
- A first step in which the magnetic nanoparticles are conjugated (attachment process) with oviduct recombinant protein (OVGP1r). Said protein is used due to its capacity to interact with the extracellular matrix, ZP, of the oocyte/embryo in a natural manner when oocytes and embryos travel through the oviduct.

Specifically, truncated recombinant protein OVGP1r, i.e., lacking region D of the C-terminal end, the region responsible for endocytosis of this protein, is used since this truncated form of OVGP1r attaches to the outer area of the ZP of the oocyte/embryo better than the whole protein does, and it is furthermore not endocytosed.

Through different experiments, it is verified that said conjugation (attachment between the nanoparticles and the OVGP1r protein) is a strong and stable attachment over time.
- A second step in which it is verified if said nanoparticle-OVGP1r conjugation, in turn, attaches to the ZP of oocytes/embryos after being incubated together.

To that end, the oocytes/embryos are co-incubated with nanoparticles-OVGP1r at different concentrations over time (up to 24 h of co-incubation).
- And once the co-incubation time has elapsed, a third step in which, after verifying the number of oocytes/embryos having nanoparticles attached to them and that they are distributed, more or less homogeneously, around the ZP without being endocytosed, it is assessed if the amount of magnetic nanoparticles attached to the ZP of oocytes/embryos is enough for said oocytes/embryos to be attracted by a magnetic field.

To that end, a magnetic device is used where it is verified that about 80% of oocytes/embryos with the nanoparticles adhered to them are attracted by the magnetic field.

It should be mentioned that preferably the diameter of the nanoparticles is comprised between 1 and 500 nanometers and that, also preferably, the nanoparticle-OVGP1r conjugation temperature is comprised between 4°C and 38°C.

In any case, this magnetic capacity is a huge breakthrough in oocyte and embryo manipulation, either for the movement of oocytes and embryos to specific locations by applying a magnetic mobile field or for keeping them immobile by means of a magnetic fixed field. There is enormous interest in the use of this technology in assisted reproduction techniques in different animal species. One of the biggest limitations in reproduction techniques consists of the actual handling and manipulation of oocytes and embryos, since they require maximum control conditions to preserve their fertilizing quality in the case of oocytes or of development in the case of embryos. Processes such as *in vitro* maturation of oocytes, artificial insemination, *in vitro* fertilization, embryonic culture and development or vitrification require the manipulation of oocytes and embryos, both for moving them and changing media and reagents, or for immobilizing them on supports which allow transfer. Moreover, the determination of the best embryonic quality for selecting the embryo to be implanted requires observing its development for several hours by means of (time-lapse) viewing techniques where the immobilization of the embryo is fundamental for the focus thereof for a long time. Finally, the magnetic nature of the particles adhered to the ZP could be used for locating the embryo in the genital tract of the female by means of magnetic resonance or other imaging diagnostic techniques.

It is important to stress what distinguishes the technique for the adhesion of the nanoparticles to the oocytes and embryos. Unlike methodologies used in oocyte labeling in which a lectin (exogenous proteins from plants) or antibody is used as an attachment element, in this case an endogenous recombinant protein from the species itself which in natural conditions is attached to the outer part of oocytes and embryos *in vivo* is used. The fact that it is a recombinant protein means that the methodology can be readily transferred to any species, because all that is required is to use the OVGP1r recombinant protein from the species itself. Moreover, this same fact means that this protein can be readily generated attached to fluorescent proteins, which will allow their easy detection.

Therefore, a second aspect of the invention relates to the use of non-human magnetic oocytes and/or embryos, prepared according to the method described above, in assisted reproduction techniques in different animal species, such as gamete manipulation or *in vitro* maturation of oocytes, artificial insemination, *in vitro* fertilization in fertility, vitrification, or culture treatments, embryonic monitoring and development, where the magnetic capacity the oocytes and/or embryos acquire with said method allows moving them to specific locations by applying a magnetic mobile field or keeping them immobile by means of a magnetic fixed field. It also allows their immobilization for viewing them by means of microscopy and imaging (time-lapse) techniques, due to the retention capacity of the magnetic field of the magnetized oocytes and/or embryos, as well as their detection, monitoring, and viewing through the female genital tract by means of magnetic resonance, due to the ferric and magnetic nature of the nanoparticles.

Given the foregoing, it can be seen that the method of preparing magnetic oocytes and/or embryos by means of nanoparticles for assisted reproduction techniques and the use of said non-human oocytes and/or embryos in assisted reproduction techniques represent an innovation having features that have been unknown up until now for the purpose for which it is intended. These reasons and the practical usefulness of the innovation provide sufficient grounds for obtaining the exclusive privilege which is sought.

### Preferred Embodiment of the Invention

Specific examples of the method of the invention applied in pig oocytes are described below.

### - Conjugation of OVGP1r with magnetic nanoparticles (MNPs):

In this experiment a model of MNPs, *Small Carboxyl-Modified Paramagnetic Microspheres* (-COOH) (Estapor®), was used. These ferric MNPs have a high magnetic capacity, with a diameter of 0.365 µm and a concentration of 1 mg/ml. For handling, a magnetic rack (GE Healthcare, Little Chalfont, United Kingdom) was used.

To conjugate OVGP1r with the MNPs, 10 µl (1 mg MNPs/ml) of MNPs were used. First, the MNPs were washed twice in 500 µl of Milli-Q water and resuspended in 240 µl of activation buffer (100 mM sodium phosphate, pH 6.2), 30 µl of 1-(3-dimethylaminopropyl)-3-ethylcarbodiimide HCl or EDC conjugation buffer (50 mg/ml diluted in water), and 30 µl of Sulfo NHS conjugation buffer (50 mg/ml diluted in water). Then they were kept under stirring for 20 minutes at room temperature. After this time, the MNPs were washed twice in 500 µl of coupling buffer (0.1 M sodium bicarbonate, pH 8) and incubated with 15 µl (corresponding to 6 µg) of OVGP1r (0.4 mg/ml) in 300 µl of coupling buffer (0.1 M sodium bicarbonate, pH 8) overnight at 4°C or at room temperature for 2 hours.

The next day (or after 2 hours), the MNPs were washed twice in PBS (20 mM sodium phosphate buffer). All the experiments were performed with a set of buffers to improve conjugation and storage conditions of the MNP-OVGP1r complex.

### - Electrophoresis and Western Blot:

For the purpose of verifying that OVGP1r-MNP conjugation had been carried out successfully, electrophoresis and Western Blot were performed.

First, the MNPs were incubated with loading buffer for 10 minutes at 100°C. Then electrophoresis was performed at 400 milliamperes and 200 volts for 40 minutes. A commercial polymerized acrylamide-bisacrylamide gel was used: NovexTM WedgeWellTM 4-20% Tris-glycine 1.0 mm x 100 Well (InvitrogenTM).

After electrophoresis, the proteins of the gel were transferred to a polyvinylidene difluoride (PVDF) membrane with a pore size of 0.45 µm (Millipore Corporation, Bedford) previously treated according to the manufacturer's instructions. The transfer was carried out in XCell II TM Blot Module (Invitrogen TM) at 30 volts and 400 milliamperes for 1 h and 6 minutes.

Once the transfer was finished, the membrane was washed three times with TBS-T (50 mM Tris, pH 7.4, 150 mM NaCl, and 0.1% Tween 20). Subsequently, the membrane was blocked with TBS-T with 1% bovine serum albumin (BSA, Sigma-Aldrich) for 1 hour under slow stirring at room temperature or at 4°C overnight. Next, the membrane was washed with TBS-T three times for 10 minutes each wash and incubated with anti-rabbit-HRP antibody (Santa Cruz Biotechnology) at a concentration of 1:40,000 for 1 hour at room temperature.

Finally, the membrane was washed three times for 10 minutes with TBS-T and developed using the Pierce ECL-Plus reagent (Thermo Scientific). To detect the protein, the ImageQuant™ LAS 500® image analyzer was used.

### - Obtaining and in vitro maturation of pig oocytes:

The oocytes used were obtained from prepubertal gilts from a slaughterhouse. The ovaries are transported to the laboratory in a sterile saline solution at 38.5°C within the first hour after the animals were sacrificed. Once in the laboratory, the ovaries were washed with hexadecyltrimethylammonium bromide (CTAB) and saline solution at 38.5°C.

To obtain oocytes, ovarian follicles sized between 3 and 6 mm were aspirated using 10 ml syringes and sterile 18x40 mm needles. The follicular fluid was deposited in 15 ml conical tubes placed on a hot plate at 38.5°C.

After 5 min, the supernatant was removed, the pellet was washed with PBS at 38.5°C and deposited in a Petri dish. The cumulus-oocyte complexes (COCs) were selected by means of aspiration, using a stereo microscope (Motic SMZ-168) and a glass Pasteur pipette elongated by heat and connected to a silicone cannula. Oocytes with a homogeneous cytoplasm and at least three layers of cells of the cumulus were selected.

The selected COCs were washed twice in warm PBS before transferring them to NCSU-37 culture medium, previously equilibrated at 38.5°C in the incubator with 5% CO₂ and a humidity-saturated atmosphere for at least 3 hours. The COCs were cultured in groups of 50-55 in a 0.5 ml culture medium volume. For the first 20-22 hours of culture, the COCs were cultured in the presence of pregnant mare serum gonadotropin (PMSG), human chorionic gonadotropin (HCG) and dibutyryl cAMP. After these initial hours, the COCs were transferred to NCSU-37 medium without hormones, where they were first washed once and then cultured for another 20-22 hours. At the end of the 40-44 hours of COC culture, they were subjected to mechanical decumulation by gentle pipetting until the cells of the cumulus were separated. Then only those oocytes without cumulus cells were selected and washed in Tyrode's albumin-lactate-pyruvate (TALP) medium.

### - Experiment 1. OVGP1r-MNP conjugation

In the first experiment, the MNPs were conjugated with OVGP1r for 2 hours at room temperature or at 4°C overnight. For handling the MNPs, it is important that the content is well mixed by means of stirring.

Next, 10 µl of MNPs were taken and mixed with 500 µl of Milli-Q water in an Eppendorf tube. The MNPs were separated from the medium using a magnetic rack (GE Healthcare). After conjugation, the total volume of conjugated MNPs (MNP-OVGP1r) was 300 µl at a final concentration of 0.033 mg of MNPs/ml.

Once conjugation ended, it was verified by means of electrophoresis and Western Blot. The analyzed fractions were: (A) 25 µl of water with 6 µg of OVGP1r, (B) 25 µl of the medium which was collected once conjugation was performed (to assess the amount of unattached protein), (C1 and C2) 25 µl of the washes that were performed after the conjugation (to assure that protein was not being lost in these washes), and (D) 25 µl of the medium with the OVGP1r that attached to the MNPs. Loading buffer (8.33 µl) was added in each fraction and incubated for 10 minutes at 100°C. Before loading the electrophoresis gel, the fractions were washed with the nanoparticles from two to four times to assure that the medium was clean.

It was also assessed if OVGP1r was still attached to the MNPs after 72 hours stored at 4°C in 20 mM sodium phosphate buffer (PBS).

### - Experiment 2. Co-incubation of MNPs OVGP1r with pig oocytes

Once the MNPs were suitably conjugated with OVGP1r protein, the *in vitro* maturation of pig oocytes was performed.

The oocytes were divided into three groups and incubated as follows:
- Control group: the oocytes were incubated with 20 µl (concentration of 1.33 µg MNPs/ml) of MNPs without conjugated OVGP1r.
- 10 µl group: the oocytes were incubated with 10 µl (0.67 µg MNPs/ml) of MNPs-OVGPlr.
- 20 µl group: the oocytes were incubated with 20 µl of MNPs-OVGPlr.

Each group was placed in a well with TALP medium and stored in the incubator at 38.5°C and 5% CO₂.

The oocytes-MNP attachment was assessed at different times: 0.5, 1, 6, and 24 h of co-incubation. After these times, some oocytes were separated from each group, washed in TALP medium, and transferred to a well with 0.5 ml of TALP medium.

The oocytes were then subjected to a magnetic field for the purpose of assessing the number of magnetized oocytes. To that end, the 0.5 ml of TALP medium with the oocytes were transferred to an Eppendorf tube which was placed in a magnetic rack. The medium was then slowly collected (with an automatic 500 µl micropipette) without removing the Eppendorf tube from the magnetic rack and was placed back in the well. The number of oocytes in the well was counted and recorded, as they represent the number of oocytes that were not retained by the magnet. Finally, the magnet was removed and the TALP medium (with the oocytes) was again passed through the Eppendorf tube in order to recover the oocytes which had attached in the presence of the magnet (referred to as the percentage of magnetized oocytes).

Having sufficiently described the nature of the present invention, as well as the manner of putting it into practice, it is not considered necessary to provide further explanation for one skilled in the art to understand the scope thereof and the advantages derived from it, and within its essential features, said invention may be carried out to practice in other embodiments differing in detail from the embodiment indicated by way of example, and such embodiments will likewise be covered under the protection that is sought provided that the fundamental principle thereof is not altered, changed, or modified.

## Claims

1. A method of preparing magnetic oocytes and/or embryos by means of nanoparticles for assisted reproduction techniques in different animal species, **characterized by** comprising the following steps:
- a first step in which magnetic nanoparticles are conjugated, by means of an attachment process, with oviduct protein (OVGP1); specifically, with OVGP1r recombinant protein;
- a second step in which, after being incubated together, it is verified that the nanoparticle-OVGP1r conjugation, in turn, attaches to the oocyte/embryo Z;
- and once the co-incubation time has elapsed, a third step in which the number of oocytes/embryos having nanoparticles attached to them is verified, and it is verified that they are distributed, more or less homogeneously, around the ZP without being endocytosed, and it is verified that the amount of magnetic nanoparticles attached to the ZP of oocytes/embryos is enough for the oocytes/embryos to be attracted by a magnetic field.

2. The method of preparing magnetic oocytes and/or embryos according to claim 1, **characterized in that** in order to verify that the nanoparticle-OVGP1r conjugation attaches to the ZP of oocytes/embryos, the oocytes/embryos are co-incubated with the nanoparticles-OVGP1r at different concentrations over a time of up to 24 hours of co-incubation.

3. The method of preparing magnetic oocytes and/or embryos according to claim 1 or 2, **characterized in that** in order to assess if the amount of magnetic nanoparticles attached to the ZP of oocytes/embryos is enough for the oocytes/embryos to be attracted, the oocytes/embryos are subjected to a magnetic field.

4. The method of preparing magnetic oocytes and/or embryos according to claim 3, **characterized in that** the amount of magnetic nanoparticles attached to the ZP of oocytes/embryos is enough for the oocytes/embryos to be attracted when 80% of oocytes/embryos with the nanoparticles adhered to them are attracted by the magnetic field.

5. The method of preparing magnetic oocytes and/or embryos according to any of claims 1 to 4, **characterized in that** the diameter of the nanoparticles is comprised between 1 and 500 nanometers.

6. The method of preparing magnetic oocytes and/or embryos according to any of claims 1 to 5, **characterized in that** the nanoparticle-OVGP1r conjugation temperature is comprised between 4°C and 38°C.

7. Use in assisted reproduction techniques of non-human oocytes and/or embryos prepared with a method such as the one described in any of claims 1 to 6.

8. Use in assisted reproduction techniques of non-human oocytes and/or embryos according to claim 7, **characterized by** comprising the gamete manipulation.

9. Use in assisted reproduction techniques of non-human oocytes and/or embryos according to claim 7, **characterized by** comprising the *in vitro* maturation of oocytes.

10. Use in assisted reproduction techniques of non-human oocytes and/or embryos according to claim 7, **characterized by** comprising artificial insemination.

11. Use in assisted reproduction techniques of non-human oocytes and/or embryos according to claim 7, **characterized by** comprising *in vitro* fertilization in fertility, vitrification, or culture treatments.

12. Use in assisted reproduction techniques of non-human oocytes and/or embryos according to claim 7, **characterized by** comprising embryonic monitoring and development.
